# EUROPEAN PATENT APPLICATION

(11) **EP 1 617 222 A1**
(43) Date of publication of application: **18.01.2006**
(21) Application number: 04723734.2
(22) Date of filing: 26.03.2004
(51) Int. Cl.: G01N 33/68

(54) **METHOD FOR COLLECTING INFORMATION ON THE PRIMARY STRUCTURE OF PROTEINS, HYDROPHOBIC MICROPARTICULATE CARRIER USED IN SAME, AND SYSTEM FOR AUTOMATICALLY OBTAINING INFORMATION ON THE PRIMARY STRUCTURE OF PROTEINS**

(30) Priority: 31.03.2003 JP 2003095904
(71) Applicant: RIKEN, Wako-shi, Saitama 351-0198 (JP)
(72) Inventor: NAKAYAMA, Hiroshi, 3510198 (JP); MIURA, Hiromi, 3510198 (JP); IWAKI, Masaya, 3510198 (JP)
(74) Representative: von Kreisler Selting Werner
(86) International application number: PCT/JP2004/004247
(87) International publication number: WO 2004/088323

(57) **Abstract**

A method for obtaining information on the primary structure of peptides comprising fragmenting the proteins, which enables automation of obtaining information on the primary structure of a peptide sample; a device for use in the method; and an automatic analysis system on the primary structure of the peptides. The method for obtaining information on the primary structure of peptides comprising the steps of: denaturing, reducing, and alkylating a protein; immobilizing the reduced alkylated protein obtained on a hydrophobic microparticulate carrier; chemically or enzymatically fragmenting the immobilized protein obtained; and obtaining information on the primary structure of the peptides fragmented. The automatic analysis system on the primary structure of the peptides comprising an automatic fragmentation element for automatically fragmenting a reduced and alkylated protein, an automatic mass spectrometry element for automatically analyzing the fragmented peptides by mass spectrometry, and a data analysis element for analyzing the data obtained by the automatic mass spectrometry element. The automatic fragmentation of the reduced and alkylated protein is conducted using a hydrophobic microparticulate carrier.

## Description

### Technical Field

The present invention relates to a method of fragmenting a protein sample adsorbed on a solid phase to produce peptides, a device for the same, and an automatic analysis system comprising that device and a device for obtaining information on the primary structure of the peptides produced.

### Background Technology

Today, as the genomes of many living organisms, including humans, are being steadily sequenced, determining the functions of the proteins encoded by these genomes is a major issue in post-genome sequencing. Determining the function of a protein requires analyzing the primary structure of the protein. Analyzing the primary structure of a protein requires cleaving the protein into peptides. However, proteins are varied and cannot necessarily be uniformly rendered into peptides at present.

Genome sequencing of many model organism species has been completed, but the reliability of the sequencing is not necessarily high. Further, it is difficult to anticipate open reading frames (the reading frames of proteins), particularly in eukaryotes. Accordingly, since analysis at the protein level permits the evaluation and correction of a genome sequence and the determination of reading frames, it is useful for obtaining information serving as the foundation of genome science.

In the analysis of the functions of genes and proteins in the post-genome sequence era, the need is increasing for analysis of the functions of proteins inside and outside cells and the systematic analysis of the functions of proteins expressed artificially in cellular systems or cell-free systems. Confirmation of whether these proteins have been synthesized as designed becomes important fundamental information for functional analysis.

This need to analyze various and numerous proteins calls for an automated analysis system, not the current labor-intensive method.

The following three points are important in the efficient degradation of proteins and the high-yield recovery of peptides:
(1) Denaturation of proteins and reduction of nonuniformity of degradation caused by the blocking of stereostructures;
(2) Reduction to sever S-S bonds within protein molecules and between molecules, and protection of the highly reactive cysteine residue SH group that is produced;
(3) The use of highly residue-specific proteases.

Thus, in protein chemistry, a protein sample is generally denatured, reduced, and alkylated, and then digested by proteases to prepare fragmented peptide samples (New Biochemical Test Methods, Protein II Primary Structures, Tokyo Kagaku Dojin, 1990).

Denaturing agents and alkylating agents block many proteases. Thus, when conducting operations in a solution, it is necessary to either:
(1) dilute the denaturing agent or alkylating agent concentration to a degree allowing the enzyme to function, or
(2) remove the denaturing agent or alkylating agent by reverse-phase liquid chromatography, dialysis, organic solvent precipitation, or the like.

However, when using these methods, the protein often becomes insoluble or tends to adhere to the vessel wall or the like when the denaturing agent is removed, so there are problems such as deterioration of the protein recovery rate and low fragmentation efficiency.

Robots that conduct various processes such as the chemical reaction, enzyme digestion, and desalting of protein samples are commercially available. These robots are basically solution-handling robots. That is, they conduct liquid phase reactions based on combinations of operations in which pipets are employed to aspirate and discharge solutions between numerous containers. Accordingly, the above-stated problems of solution reactions have not been solved.

A method of adsorbing a protein onto a cellulose film or polyvinylidene difluoride film and chemically or enzymatically fragmenting the protein on the film has been reported (Aebersold R.H., Leavitt J., Saavedra R.A., Hood L.E., Kent S.B., Proc. Natl. Acad. Sci. USA, Oct.; 84(20): 6970-4).

In this method, a protein is immobilized on a film by hydrophobic interaction, various reactions are conducted on the film, the excess reagent or the like is removed, and the protein is chemically or enzymatically fragmented. However, in this method, handling of the film is a tedious manual operation requiring skill.

Since the protein fragmentation process is tedious and requires a skill dependent on the properties of individual proteins, no general automated method has been established.

Accordingly, the present invention has for its object to provide a method for collecting information on the primary structure of a protein comprising a protein fragmentation method permitting automation of the acquisition of information on primary structure from a protein sample.

The present invention further provides a device employed in this method and a system for automatically obtaining information on the primary structure of a protein sample.

### Description of the Invention

In the present invention, various investigations were conducted to solve the above-stated problems. It was discovered that by immobilizing on a solid phase the enzymatically digested portion in the conventional method, it was possible to automatically obtain information on primary structure from the protein sample; the present invention was devised on that basis. That is, in the present invention, various operations are conducted in the protein fragmentation process by immobilizing a protein on a hydrophobic microparticulate carrier, permitting the automation of acquiring primary structural information from a protein sample.

The present invention relates to a method for collecting information on the primary structure of a protein comprising:
step (1) of denaturing, reducing, and alkylating a protein;
step (2) of immobilizing the reduced alkylated protein obtained in step (1) on a hydrophobic microparticulate carrier;
step(3) of chemically or enzymatically fragmenting the immobilized protein obtained in step (2); and
obtaining information on the primary structure of the peptides fragmented in step (3).

The above method for collecting information on the primary structure of a protein of the present invention desirably further comprises step (4) of recovering the fragmented peptides obtained in step (3) from the carrier, step (5) of isolating the fragmented peptides recovered in step (4), and obtaining information on the primary structure of the peptides isolated in step (5).

It is also desirable that the hydrophobic microparticulate carrier is glass, silica, or an organic polymer having a hydrophobic surface, and the hydrophobic microparticulate carrier has an average particle diameter falling within a range of 1 to 50 micrometers.

The present invention further relates to a hydrophobic microparticulate carrier for use in immobilizing reduced and alkylated protein, fragmenting the immobilized, reduced and alkylated protein, and recovering the fragmented peptides that are generated.

In the above-described hydrophobic microparticulate carrier of the present invention, the hydrophobic microparticulate carrier is desirably glass, silica, or an organic polymer having a hydrophobic surface, and
the hydrophobic microparticulate carrier desirably has an average particle diameter falling within a range of 1 to 50 micrometers.

The present invention further relates to a system for automatically obtaining information on the primary structure of a protein comprising an automatic fragmentation element for automatically fragmenting a reduced and alkylated protein, an automatic mass spectrometry element for automatically analyzing the fragmented peptides by mass spectrometry, and a data analysis element for analyzing the data obtained by the automatic mass spectrometry element, characterized in that the automatic fragmentation of the reduced and alkylated protein is conducted using the hydrophobic microparticulate carrier of the present invention.

In the above system, the automatic fragmentation element desirably comprises a container filled with sample protein solution, a container filled with a wash solution, a container filled with an enzyme solution, a container filled with eluate, a pipet tip on which is held the hydrophobic microparticulate carrier of the present invention, a pipet for aspirating and discharging liquids in the containers into and out of the pipet tip, a thermostatic vat into which the pipet tip can be placed in a state of constant temperature, and an XYZ arm for moving the pipet. The system also desirably comprises a container for receiving eluting fragmented peptides.

In the above system, the automatic fragmentation element desirably comprises a container filled with a sample protein solution, a container filled with a wash solution, a container filled with an enzyme solution, a container filled with eluate, a column holding the hydrophobic microparticulate carrier of the present invention, a thermostatic vat into which the column can be placed in a state of constant temperature, and a pump for sending solution to the column from the above containers.

In the above system, the automatic mass spectrometry element desirably comprises a matrix-supported laser desorption ionization/flight time mass spectrometer or electrospray ionization/mass spectrometer, the data analysis element desirably comprises search engine software employing mass data to search an ordered database and identify peptides, and also comprises hardware performing computations relating to this software and data.

### Brief Description of the Drawings

Fig. 1 is a drawing descriptive of a fragmented peptide producing device employing pipet tips holding a hydrophobic microparticulate carrier.
Fig. 2 is a drawing descriptive of a fragmented peptide producing device employing columns holding a hydrophobic microparticulate carrier.
Fig. 3 is a base peak chromatogram of bovine serum albumin fragmented by trypsin on a hydrophobic microparticulate carrier.

### Best Mode of Implementing the Invention

### (Method for collecting information on the primary structure of a peptide)

### Step (1)

In step (1), a protein is denatured, reduced, and alkylated. The reduction and alkylation of the protein can be conducted using known methods without modification.

For example, denaturation can be conducted with guanidine hydrochloride or urea.

Reduction can be conducted with thiols such as dithiothreitol and phosphines such as tributylphosphine.

Alkylation can be conducted with iodoacetic acid, iodoacetamide, 4-vinyl pyridine, acrylamide, ethylene imine, and the like.

The protein supplied in the method of the present invention is not specifically limited; the method of the present invention can be applied to any protein.

Examples of such proteins are: soluble proteins, membrane proteins, fiber proteins, glycoproteins, protein complexes, and complexes of proteins and nucleic acids.

### Step (2)

In step (2), the reduced and alkylated protein obtained in step (1) is immobilized on a hydrophobic microparticulate carrier.

The hydrophobic microparticulate carrier can be glass, silica, or an organic polymer having a hydrophobic surface. Specific examples of glass, silica, and organic polymers having hydrophobic surfaces are glass, silica, and organic polymers to which are bonded straight-chain alkyl groups such as methyl, ethyl, propyl, butyl, octyl, and octadecyl groups or aromatic functional groups such as phenyl groups. Examples of organic polymers are polystyrene, styrene divinylbenzene polymer, and polyvinyl alcohol.

The hydrophobic microparticulate carrier can have an average particle diameter falling within a range of 1 to 50 micrometers.

The reduced and alkylated protein can be immobilized on the hydrophobic microparticulate carrier by the following methods, for example.

The immobilization method can be for the most part prescribed by the holding container. For example, when employing a pipet column, immobilization can be conducted by repeatedly aspirating and discharging a protein sample with a pipet (the "Pipetoman" from Gilson Corp.). When holding the sample in a small column (made of stainless steel or the like) used for on-line treatment in liquid chromatography, the protein can be immobilized by introducing the sample into the column by delivering the fluid by means of the pump of a chromatographic device together with injection from a syringe.

In the protein fragmentation method of the present invention, a hydrophobic microparticulate carrier is employed instead of the above-described membrane as a carrier that is independent of the shape of the holding container or the like and is readily handled.

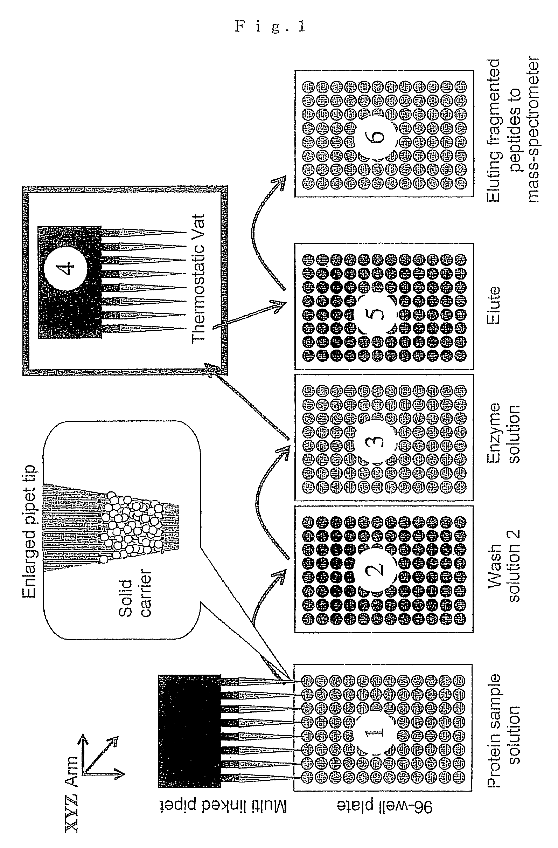

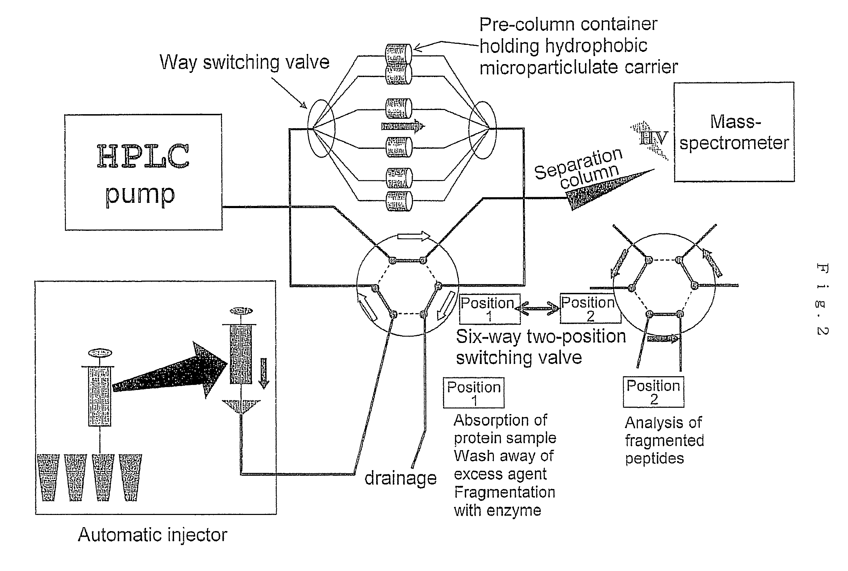

By immobilizing the protein on the hydrophobic microparticulate carrier, substances that impede enzyme activity and mass spectrometry can be readily washed away in the same manner as in the above membrane method. In conventional methods, this operation is conducted by manually removing and introducing solutions in containers. By contrast, according to the present invention by using a hydrophilic microparticulate carrier held in a container such as a pipet or precolumn, this operation can be readily incorporated into an automatic solution delivery system (see Fig. 2) having a solution handling robot (see Fig. 1) or electric valves. Accordingly, steps requiring tedious manual labor in the conventional method can be automated.

### Step (3)

In step (3), the immobilized protein obtained in step (2) is chemically or enzymatically fragmented.

For example, the immobilized protein can be chemically fragmented by the following methods: acid hydrolysis with dilute hydrochloric acid (preferentially cleaves the carboxyl group side of aspartic acid), cyanogen bromide degradation (methionine-specific), and oxidative bromination (tryptophan-specific cleavage using 2-(2-nitrophenylsulphenyl)-3-methyl-3-bromoindole and the like).

The immobilized protein can be enzymatically fragmented using a variety of enzymes. For example, trypsin, lysine endopeptidases, V8 protease, and other highly substrate-specific enzymes are often employed, but any enzyme capable of protein fragmentation may be employed.

The method of the present invention may further comprise steps (4) and (5) following steps (1) to (3).

### Step (4)

In step (4), the fragmented peptides obtained in step (3) are recovered from the carrier. The fragmented peptides may be recovered from the carrier in the following manner, for example.

The fragmented peptides may be recovered by exposing the carrier to an aqueous solution of a polar organic solvent such as acetonitrile, methanol, or 2-propanol.

### Step (5)

In step (5), the fragmented peptides recovered in step (4) are isolated. The fragmented peptides that have been recovered may be isolated in the following manner, for example. They may be isolated by reverse-phase liquid chromatography employing a column packed with hydrocarbon chemically-bonded silica. A concentration gradient elution method based on a mobile phase comprised of a mixture of two solutions in the form of an aqueous solution of trifluoroacetic acid, formic acid, or hexafluorobutyric acid, and a polar organic solvent solution such as acetonitrile, methanol, 2-propanol, or the like containing trifluoroacetic acid, formic acid, or hexafluorobutyric acid may be employed.

Information on primary structure may be obtained from the peptides that are fragmented in step (3) or the peptides that are isolated in step (5). Information on primary structure may be obtained as follows, for example.
(1) The Edman method, in which amino acids are sequentially cleaved at the amino-terminus of the protein or peptide one residue at a time with phenyl isothiocyanate and the phenyl thiohydantoin-amino acid product is identified.
(2) Obtaining mass information about the peptide by mass spectrometry and looking it up in a sequence database. A; In the case of a single or simple mixture, a sequence database can be searched based on a set of molecular weight information on the peptides and the original protein identified. B; Peptides can be isolated in a mass spectrometer, cleaved by collision with the molecules of a rare gas such as argon or helium, and the ion spectra obtained can be used to search a sequence database to identify the original protein.
(3) When the protein itself has been identified and modified structure information is being sought, the molecular weight of the peptides can be obtained by mass spectrometry to detect the modified structure.

The present invention covers the hydrophobic microparticulate carrier employed in immobilizing a reduced and alkylated protein, fragmenting the immobilized reduced and alkylated protein, and recovering the resulting fragmented peptides. As set forth above, an example of this carrier is a hydrophobic microparticulate carrier having an average particle diameter of 1 to 50 micrometers in the form of glass, silica, or an organic polymer having a hydrophobic surface.

The hydrophobic microparticulate carrier of the present invention can be held in a container, such as a pipet tip or column, capable of aspirating and discharging liquids.

The present invention covers a system for automatically obtaining information on the primary structure of a protein comprising: an automatic fragmentation element for automatically fragmenting a reduced and alkylated protein, an automatic mass spectrometry element for automatically analyzing the fragmented peptides by mass spectrometry, and a data analysis element for analyzing the data obtained by the automatic mass spectrometry element. This system is characterized in that automatic fragmentation of the reduced and alkylated protein is conducted using the above-described hydrophobic microparticulate carrier of the present invention.

More specifically, the automatic fragmentation element may, for example, comprise a container filled with a sample protein solution, a container filled with a wash solution, a container filled with an enzyme solution, a container filled with eluate, a pipet tip on which is held the hydrophobic microparticulate carrier of the present invention, a pipet for aspirating and discharging liquids in the container onto the pipet tip, a thermostatic vat into which the pipet tip can be placed in a state of constant temperature, and an XYZ arm for moving the pipet. The automatic fragmentation element may further comprise a container for receiving eluting fragmented peptides.

The automatic fragmentation element may comprise a container filled with a sample protein solution, a container filled with a wash solution, a container filled with an enzyme solution, a container filled with eluate, a column on which is held the hydrophobic microparticulate carrier of the present invention, a thermostatic vat into which the column can be placed in a state of constant temperature, and a pump for transferring solution from the above containers to the column.

The automatic mass spectrometry element may comprise an automatic injector, liquid chromatograph and isolation column, and mass spectrometer, for example. The mass spectrometer, for example, may be a matrix supported laser desorption ionization/flight time mass spectrometer or electrospray ionization/mass spectrometer. Examples of mass spectrometers that can be combined with the electrospray ionization method for use are quadrupole, ion-trap, flight time, Fourier transform ion cyclotron, magnetic, and hybrid devices.

The data analysis element is comprised of search engine software for searching a sequence database with mass data to identify a peptide, a sequence database, and hardware performing computations relating to this software and data.

The above containers filled with various liquids may be multiwell plates comprising multiple containers. Further, a single device may have multiple pipets. The multiple pipets may be simultaneously moved by the XYZ arm. And the aspiration and discharge of solutions, and their placement in a state of constant temperature, may be simultaneously conducted by pipet tips mounted on the multiple pipets.

The system employing pipet tips holding hydrophobic microparticulate carriers will be described next based on Fig. 1.

This system employs solution-handling robots equipped with hydrophobic microparticulate carrier holding pipet tips, XYZ arms, and multiple linked pipets.

Fig. 1 describes the case where a 96-well plate is employed as the container filled with various solutions and multiple linked pipets capable of simultaneously handling eight pipet tips are employed. Fig. 1 shows a container filled with a sample protein solution, a container filled with a wash solution, a container filled with an enzyme solution, a container filled with eluate, a pipet tip on which is held the hydrophobic microparticulate carrier, a thermostatic vat into which the pipet tip can be placed in a state of constant temperature, and an XYZ arm for moving the pipet tip. The operation of this device will be described below.
1) The protein test solution is denatured, reduced, and alkylated by the solution-handling robot (not shown).
2) The reduced and alkylated protein solution is aspirated into the pipet that holds the hydrophobic microparticulate carrier and the protein is immobilized on the surface of the microparticles. (1)
3) The reagent employed in denaturation, reduction, and alkylation is washed away by aspirating and discharging buffer solution with the pipet. (2)
4) A blocking solution is aspirated and discharged to mask hydrophobic adsorption points. (2) This step may be omitted.
5) Excess blocking reagent is washed away by aspirating and discharging buffer solution with the pipet. This step may also be omitted. (2)
6) Protease solution is aspirated with the pipet. (3)
7) The pipet tip is detached from the pipet in a thermostatic chamber and an enzymatic reaction is conducted for several hours. (4)
8) A new pipet tip is loaded onto the pipet and the above steps are repeated from the protein adsorption in 1).
9) When the enzymatic reaction time has elapsed, the pipet tip in the thermostatic chamber is reloaded onto the pipet, and organic solvent (acetonitrile, methanol)/aqueous solution is aspirated and discharged to elute the peptides that have been produced into the container. (5)(6)
10) The organic solvent concentration in the eluate is evaporated by blowing nitrogen gas, thereby reducing the concentration of organic solvent in the solution. (6)
11) The eluate is injected into a high-performance chromatography ― mass spectrometer device (not shown) by an automatic injection device, for example.
12) The high performance chromatography - mass spectrometer device separates the peptides and measures the molecular weight of each peptide (not shown).

The system employing a column to hold the hydrophobic microparticulate carrier will be described next based on Fig. 2.

The system of Fig. 2 comprises a precolumn container holding hydrophobic microparticulate carrier and an automatic solution transfer system.

In Fig. 2, an automatic injector supplies a column packed with hydrophobic microparticulate carrier with various solutions from a container filled with a sample protein solution, a container filled with a wash solution, a container filled with an enzyme solution, or a container filled with eluate. This column is positioned in a thermostatic vat. Each column is connected to or cut off from the automatic injector by a flow route switching valve. Targeted solutions are fed and discharged by the automatic injector. In the end, the fragmented peptides are connected in tandem to a column holding the fragmented peptides and the separation column by switching a six-way, two-position valve to position 2. The concentration of organic solvent is increased by a high-pressure mixing method employing two solution transfer devices (pumps), thereby causing the peptides to elute out and separate from the columns connected in tandem and be introduced on-line to the mass spectrometer.

The operation of this device will be described below.
1) A protein sample solution is denatured, reduced, and alkylated by a sample-handling robot.
2) A six-way, two-position electric valve is switched to position 1 in advance. The solution of reduced and alkylated protein is injected into a liquid transfer system employing an automatic injector and passed into a hydrophobic microparticulate carrier-holding precolumn that has been pre-equilibrated with buffer solution.
3) A blocking reagent solution is injected by the automatic injector. This step may be omitted.
4) After the automatic injector injects a protease solution, a flow path switching valve is switched to cut the precolumn off from the liquid supply.
5) An enzymatic reaction is conducted for several hours. During the enzymatic reaction, 2) to 4) are conducted by the next precolumn.
6) When the enzymatic reaction ends, the six-way, two-position electric valve is switched to position 2, connecting to the high-performance chromatography - mass spectrometry device.
7) The high-performance chromatography - mass spectrometry device separates the peptides and measures the molecular weight of individual peptides.

In the device collecting information on the primary structure of peptides, the unit obtaining information on primary structure from the fragmented peptides, for example, can be a mass spectrometer. An automated version of the Edman method is a further example.

### Examples

The present invention is described in greater detail below based on examples.

The device shown in Fig. 1 was employed using a pipet-type column, Porostip R2 (Applied Biosystems Corp.) in which PorosR2 (particle diameter of 20 micrometers) from Applied Biosystems Corp. was held as the hydrophobic microparticulate carrier. The digestion of a protein with trypsin on these hydrophobic beads will be described below.

### 1. Reagents and products

### 1-1. 96-well plate

- Plate 1: V-bottom plate (for small quantities of reagent and sample, ABgene Corp. ThermoFast96, skirted)
- Plate 2: Deep-bottom plate (for 100 microliters or more of wash solution)

### 1-2. Storage solution

When no reagent has been specified, the reagent on hand with the highest purity is employed. When employing water, only ultrapure water is employed.
- Equilibration solution:: 0.085 percent trifluoroacetic acid aqueous solution
- Eluting solution:: 50 percent acetonitrile/0.085 percent trifluoroacetic acid
- Wash solution 1:: Acetonitrile (high-performance liquid chromatography grade)
- Wash solution 2:: 50 mM Tris-HCl buffer solution (pH 8.5)

### 1-3. Solutions prepared during use

- Enzyme solution:: TPCK-treated trypsin (modified trypsin from Promega Corp. or the like) dissolved to 1 to 8 pmol/microliter in 20 mM acetic acid.
- Protein solution:: Bovine serum albumin (final concentration 1 to 4 pmol/microliter, each well containing about 50 microliter) denatured with 6 M of guanidine hydrochloride by a solution-handling robot in a V-bottom 96-well plate, reduced by dithiothreitol, and alkylated with iodoacetic acid (carboxymethylation) was employed.

### 2. Preparation operations

### 2-1. Positioning of 96-well plates and injection of reagents

A plate containing protein reagent, two new V-bottom plates, and three deep-bottom plates were placed in prescribed positions (see Fig. 1, the wash solution plate is not shown). A 200 microliter pipet tip and a PorostipR2 pipet column were positioned on the pipet rack.
The following injection operations were performed by the solution-handling robot.
A 400 microliter quantity of wash solution 1 was injected into each well of plate 1.
A 400 microliter quantity of wash solution 1 was injected into each well of plate 2.
A 10 microliter quantity of enzyme solution was injected into each well of plate.
A 40 microliter quantity of eluate was injected into each well of plate.

### 2-2. Pretreatment

Porostips R2 were mounted on an 8-bundle pipet and the following operations were conducted.
A 100 microliter quantity of wash solution 1 was aspirated and discharged into a waste solution sink. This was repeated twice.
A 100 microliter quantity of wash solution 2 was aspirated and discharged into a waste solution sink. This was repeated twice.

### 3. Reagent protein adsorption and washing away of foreign matter such as denaturing agent

The reagent solution was aspirated and discharged. This was repeated 10 times.
A 100 microliter quantity of wash solution 2 was aspirated and then discharged into a waste solution sink. This was repeated three times.

### 4. Enzymatic fragmentation

A 10 microliter quantity of enzyme solution was aspirated and discharged into buffer solution. After repeating this aspiration and discharge twice, enzyme solution was again aspirated.

In this aspirated state, the pipet was detached over a 96-well plate prepared in a 37°C thermostatic chamber. The machine went on standby for 3 hours. (When the solution contains more than eight specimens, 2-2. to 4 is repeated.)

### 5. Recovery of fragmented peptides

When the time had elapsed, the pipet was installed and the enzyme solution was discharged to a 96-well recovery-use plate. A 40 microliter quantity of the injected eluate was aspirated; two aspirations and discharges were made at that spot. A further aspiration was then made and discharged into a 96-well recovery-use plate.

Nitrogen gas was blown into each well of the 96-well recovery-use plate, volatizing the acetonitrile and concentrating the eluate to about 10 microliters (the flow rate of the nitrogen gas was set in advance to a rate capable of effecting concentration in about 10 minutes).

### 6. High performance chromatography - mass spectrometry

The peptide sample was injected by an automatic injector and HPLC-MS/MS analysis was conducted. The results were looked up in a database and analyzed.

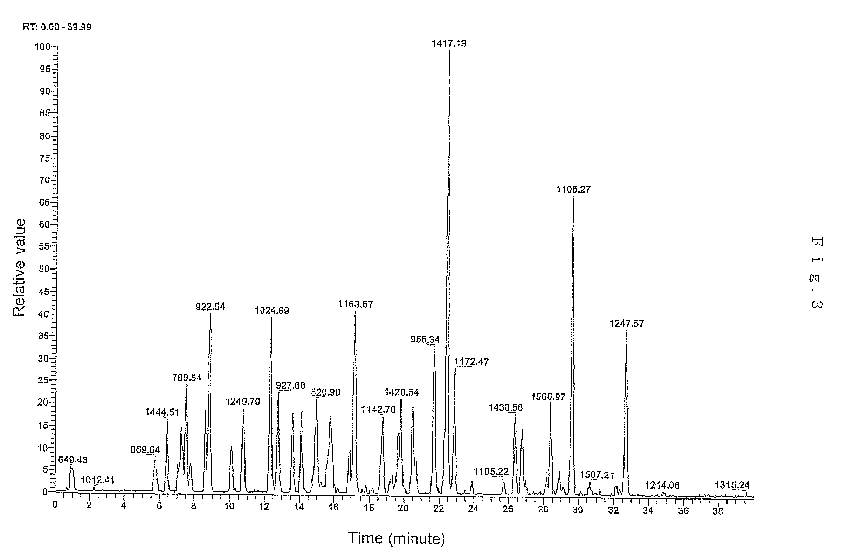

Fig. 3 shows the base peak chromatogram obtained by fragmenting bovine serum albumin by the above-described operating method.

### Industrial Applicability

The present invention permits automation in obtaining primary structure information from protein samples. Application to the functional analysis of proteins inside and outside cells and the systematic functional analysis of artificially expressed proteins in cell systems or noncellular systems can be anticipated.

## Claims

1. A method for collecting information on the primary structure of a protein comprising:
step (1) of denaturing, reducing, and alkylating a protein;
step (2) of immobilizing the reduced alkylated protein obtained in step (1) on a hydrophobic microparticulate carrier;
step (3) of chemically or enzymatically fragmenting the immobilized protein obtained in step (2); and
obtaining information on the primary structure of the peptides fragmented in step (3).

2. The method of claim 1, further comprises step (4) of recovering the fragmented peptides obtained in step (3) from the carrier, step (5) of isolating the fragmented peptides recovered in step (4), and obtaining information on the primary structure of the peptides isolated in step (5).

3. The method of claim 1 or 2, wherein the hydrophobic microparticulate carrier is glass, silica, or an organic polymer having a hydrophobic surface.

4. The method of any of claims 1 to 3, wherein the hydrophobic microparticulate carrier has an average particle diameter falling within a range of 1 to 50 micrometers.

5. A hydrophobic microparticulate carrier for use in immobilizing reduced and alkylated protein, fragmenting the immobilized, reduced and alkylated protein, and recovering the fragmented peptides that are generated.

6. The hydrophobic microparticulate carrier of claim 5, wherein the hydrophobic microparticulate carrier is glass, silica, or an organic polymer having a hydrophobic surface.

7. The hydrophobic microparticulate carrier of claim 5 or 6, wherein the hydrophobic microparticulate carrier has an average particle diameter falling within a range of 1 to 50 micrometers.

8. A system for automatically obtaining information on the primary structure of a protein comprising an automatic fragmentation element for automatically fragmenting a reduced and alkylated protein, an automatic mass spectrometry element for automatically analyzing the fragmented peptides by mass spectrometry, and a data analysis element for analyzing the data obtained by the automatic mass spectrometry element, **characterized in that** the automatic fragmentation of the reduced and alkylated protein is conducted using the hydrophobic microparticulate carrier of any of claims 4 to 6.

9. The system of claim 8, wherein the automatic fragmentation element comprises a container filled with sample protein solution, a container filled with a wash solution, a container filled with an enzyme solution, a container filled with eluate, a pipet tip on which the hydrophobic microparticulate carrier of any of claims 4 to 6 is held, a pipet for aspirating and discharging liquids in the containers into and out of the pipet tip, a thermostatic vat into which the pipet tip is placed in a state of constant temperature, and an XYZ arm for moving the pipet.

10. The system of claim 9, wherein the system further comprises a container for receiving eluting fragmented peptides.

11. The system of claim 8, wherein the automatic fragmentation element comprises a container filled with a sample protein solution, a container filled with a wash solution, a container filled with an enzyme solution, a container filled with eluate, a column in which the hydrophobic microparticulate carrier of any of claims 4 to 6 is held, a thermostatic vat into which the column is placed in a state of constant temperature, and a pump for sending solution to the column from the above containers.

12. The system of any of claims 8 to 11, wherein the automatic mass spectrometry element comprises a matrix-supported laser desorption ionization/flight time mass spectrometer or electrospray ionization/mass spectrometer.

13. The system of any of claims 8 to 12, wherein the data analysis element comprises search engine software employing mass data to search an ordered database and identify peptides, and also comprises hardware performing computations relating to this software and data.
